(19) Europäisches Patentamt
European Patent Office
Office européen des brevets

(11) **EP 2 513 632 B1**

(12) **FASCICULE DE BREVET EUROPEEN**

(45) Date de publication et mention de la délivrance du brevet:
**18.02.2015 Bulletin 2015/08**

(21) Numéro de dépôt: **10805253.1**

(22) Date de dépôt: **08.12.2010**

(51) Int Cl.:
**G01N 21/64** *(2006.01)*     **G01N 33/02** *(2006.01)*

(86) Numéro de dépôt international:
**PCT/FR2010/000826**

(87) Numéro de publication internationale:
**WO 2011/080408 (07.07.2011 Gazette 2011/27)**

(54) **PROCEDE ET APPAREIL D'ANALYSE SPECTROSCOPIQUE, EN PARTICULIER D'ALIMENTS, UTILISANT UN TRAITEMENT MULTIVOIES DES DONNEES SPECTRALES.**

VERFAHREN UND SPEKTROSKOPISCHER ANALYSATOR, IM BESONDEREN ZUR UNTERSUCHUNG VON LEBENSMITTELN MIT MEHRKANAL-BEHANDLUNG VON SPEKTRALDATEN

METHOD AND SPECTROSCOPIC ANALYSIS APPLIANCE, ESPECIALLY FOR ANALYSING FOOD, WITH MULTI-CHANNEL TREATMENT OF SPECTRAL DATA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.03.2010 FR 1001221**
**16.12.2009 FR 0906088**

(43) Date de publication de la demande:
**24.10.2012 Bulletin 2012/43**

(73) Titulaire: **Spectralys Innovation**
**93230 Romainville (FR)**

(72) Inventeurs:
• **RIZKALLAH, Jad**
**Beyrouth (LB)**
• **BIRLOUEZ-ARAGON, Inès**
**F-95120 Ermont (FR)**

(74) Mandataire: **Reboussin, Yohann Mickaël Noël et al**
**Cabinet Orès**
**36, rue de St Pétersbourg**
**75008 Paris (FR)**

(56) Documents cités:
**WO-A2-2008/012706**

• **CHRISTENSEN J ET AL: "Fluorescence spectroscopy and PARAFAC in the analysis of yogurt", CHEMOMETRICS AND INTELLIGENT LABORATORY SYSTEMS, ELSEVIER SCIENCE PUBLISHERS B.V. AMSTERDAM, NL, vol. 75, no. 2, 28 février 2005 (2005-02-28), pages 201-208, XP025287573, ISSN: 0169-7439 [extrait le 2005-02-28]**
• **MICHELLE L. NAHORNIAK ET AL: "Optimizing the implementation of the PARAFAC method for near-real time calibration of excitation-emission fluorescence analysis", JOURNAL OF CHEMOMETRICS, vol. 17, no. 11, 1 novembre 2003 (2003-11-01), pages 608-617, XP55013827, ISSN: 0886-9383, DOI: 10.1002/cem.829 cité dans la demande**

EP 2 513 632 B1

**Description**

L'invention porte sur un procédé et un appareil d'analyse spectroscopique d'au moins un échantillon, en particulier d'un aliment ou médicament, mettant en oeuvre une méthode d'analyse des données spectroscopiques basée sur un modèle statistique multivoies. L'invention peut être appliquée en particulier, mais pas uniquement, à l'étude de l'évolution des propriétés nutritionnelles (teneur en vitamines...) et/ou toxicologiques (teneur en contaminants néoformés...) d'un aliment au cours de sa préparation ou conservation. Plus généralement, l'invention peut être appliquée à la détermination de tout indicateur de qualité d'un échantillon, et/ou de tout paramètre caractérisant un procédé auquel ledit échantillon a été soumis.

Les contaminants néoformés (CNF) sont des molécules nouvelles formées dans un aliment à partir des composants propres de cet aliment, du fait de l'action des procédés de transformation, et qui posent des problèmes de santé publique. Peu de laboratoires publics ou privés sont capables aujourd'hui de doser ces contaminants. De plus, si plusieurs méthodes ont été publiées pour le dosage de chacun des contaminants, aucune d'elles n'a réellement fait l'objet d'une standardisation et normalisation. En outre, ces analyses sont couteuses et le délai d'obtention des résultats est long (2 à 3 semaines). L'industrie nécessite des méthodes d'analyses plus rapides et moins couteuses pour assurer un contrôle de la conformité de ses procédés et de la sécurité des aliments qu'elle produit.

Les procédés de transformation alimentaire (cuisson, stérilisation, conservation) peuvent aussi influencer défavorablement les propriétés nutritionnelles des aliments, par exemple en diminuant leur taux de vitamines. La caractérisation de ces procédés et de leur influence sur les propriétés nutritionnelles des aliments pose des problèmes analogues à ceux, précités, du dosage des contaminants néoformés.

Il est connu de réaliser des analyses, notamment spectroscopiques, des aliments en ayant recours aux méthodes de la chimiométrie, et en particulier à l'analyse multivoies. L'analyse multivoies est l'extension naturelle de l'analyse multivariée lorsque les données sont arrangées dans des tableaux à trois voies ou plus. Elle se base sur l'utilisation de modèles statistiques telles que « PARAFAC » (« Parallel Factor», c'est à dire modèle à facteurs parallèles) et NPLS (« N-ways Partial Least Squares régression », c'est à dire régression par moindres carrés partiels à n-voies). Ces méthodes, ainsi que leur application à l'analyse de produits alimentaires, sont décrites dans le document [Bro 1998].

Plus précisément, [Rizkallah 2007] décrit une méthode d'analyse d'échantillons non préparés (seulement broyés, si nécessaire) basée sur l'application du modèle PARAFAC à des spectres de fluorescence frontale (« front-face fluorescence »). Cette méthode comporte l'éclairage d'un échantillon par une pluralité de rayonnements lumineux monochromatiques à des longueurs d'ondes respectives, et l'acquisition des spectres de fluorescence correspondants. Les rayonnements d'excitation échantillonnent finement (plusieurs centaines de points) une plage spectrale couvrant le visible et le proche UV ; à leur tour, les spectres d'émission de fluorescence sont échantillonnés spectralement (également sur plusieurs centaines de points). Les données spectrales ainsi recueillies pour chaque échantillon sont agencées en une matrice de grandes dimensions dite « matrice excitation-émission » (MEE), dont une dimension représente les longueurs d'onde d'émission et l'autre dimension représente les longueurs d'onde d'émission.

Les MEE correspondant à plusieurs échantillons d'étalonnage sont analysées par la méthode PARAFAC qui permet d'extraire des informations, appelées « facteurs PARAFAC » qui correspondent aux profils bilinéaires de fluorescence et à leurs intensités relatives. Puis, une régression multiple entre les intensités de fluorescence et les concentrations de contaminants néoformés (ou autres substances d'intérêt) mesurées chimiquement permet de construire un modèle d'étalonnage qui est utilisé ensuite pour prédire les concentrations de contaminants néoformés à partir des MEE recueillies sur de nouveaux échantillons à analyser.

Cette méthode présente deux limites majeures :

- Premièrement, elle nécessite un équipement de coût élevé : un spectrofluorimètre équipé d'une lampe Xénon délivrant l'ensemble des longueurs d'onde de l'UV proche au visible, et deux monochromateurs capables de faire défiler ces différentes longueurs d'onde à l'excitation comme à l'émission pour les photons émis par l'échantillon.
- Deuxièmement, sa mise en oeuvre est longue car, même si on évite la préparation de l'échantillon, l'acquisition de chaque EEM prend 15 à 45 min selon la résolution spectrale recherchée. L'analyse de ces matrices de données, comprenant un nombre considérable de variables, est également très lourde malgré l'automatisation des algorithmes de traitement des données.

Des méthodes similaires sont décrites par [Rizkallah et al., 2008] et [Christensen et al., 2005].

Le document [Nahorniak 2003] étudie l'influence de la résolution spectrale (et donc de la taille de la matrice EEM) sur l'erreur de prédiction de la concentration d'un fluorophore par application de la méthode PARAFAC. Dans ce document on considère des solutions diluées et de composition simple et connue ; en outre, toutes les mesures sont effectuées avec une résolution spectrale élevée, de 2 nm/pixel, la réduction de résolution étant obtenue en calculant des moyennes spectrales.

Il résulte de cette étude que l'erreur de prédiction est minimisée - et vaut environ 0,5-1% - pour une résolution en excitation de 6 à 10 nm/pixel, puis augmente fortement pour des résolutions inférieures (augmentation d'un facteur 2 à 6 en passant à une résolution de 20 nm/pixel). Il semble donc difficile d'utiliser des résolutions inférieures à 20 nm/pixel.

L'invention vise à procurer une méthode d'analyse plus simple, plus rapide, nécessitant un équipement plus simple et moins couteux, et par conséquent mieux adaptée aux exigences industrielles.

Conformément à l'invention un tel but est atteint par un procédé d'analyse spectroscopique selon la revendication 1.

Avantageusement, le nombre de rayonnements lumineux d'excitation, et de spectres de fluorescence correspondants pour chaque échantillon, peut être compris entre deux et six, et de préférence entre trois et cinq, sur une plage spectrale d'au moins 100 nm, et de préférence d'au moins 150 nm.

Cette méthode est innovante du fait qu'elle applique des outils d'analyse multivoies comme le modèle PARAFAC, normalement utilisés pour des MEE de larges dimensions, à un ensemble constitué par un nombre limité de spectres (généralement deux à six, de préférence entre trois et cinq), échantillonnant de manière très grossière la plage spectrale d'excitation des échantillons. Cette plage est relativement large (au moins 100, voire 150 nm, ou plus) parce que la composition physico-chimique desdits échantillons est largement inconnue, donc la position exacte des spectres d'absorption des fluorophores contenus dans le dit échantillon l'est aussi.

Les présents inventeurs ont découvert, de manière surprenante, que les modèles multivoies continuent à être applicables et fournissent des résultats exploitables même en utilisant une résolution spectrale en excitation aussi grossière, et un nombre aussi restreint de longueurs d'onde d'excitation.

Il importe de souligner que d'un point de vue physique, les *« loadings »* représentent les spectres d'émission ou d'excitation de chaque facteur, et les *« scores »* les intensités desdits facteurs. Un « facteur PARAFAC » représente la contribution d'un fluorophore, ou d'un mélange de fluorophores, commune aux échantillons analysés. Il faut aussi souligner que les spectres de fluorescence sont déformés par l'interaction entre les photons d'excitation et les photons émis avec la matrice alimentaire et que la condition de trilinéarité généralement admise pour l'application du modèle PARAFAC n'est pas satisfaite. Néanmoins, les inventeurs ont montré que ce modèle est utilement appliqué à de telles matrices EEM pour en décrire la composition en fluorophores.

Il faut bien comprendre que le procédé de l'invention ne vise pas (au moins: pas nécesairement) à déterminer la teneur des fluorophores eux-mêmes. Ce procédé exploite simplement des corrélations, en général non-linéaires ou multilinéaires, entre les évolutions des spectres d'excitation et d'émission de fluorescence et certains paramètres de qualité de l'échantillon (teneur en nutriments et/ou contaminants qui, le plus souvent, ne sont pas fluorescents ; charge microbienne...) ou certains paramètres caractérisant un procédé auquel ledit échantillon a été soumis (valeur stérilisatrice, pasteurisatrice ou cuisatrice, ou un produit temps-température, par exemple).

Une comparaison avec le document précité [Nahorniak 2003] est particulièrement intéressante. Ce document se rapporte à un cas particulièrement favorable, à savoir l'analyse de solutions de composition simple et connue. Au contraire, l'invention se rapporte à l'analyse par fluorescence frontale d'échantillons complexes et fortement diffusants, dont la composition est largement inconnue. En outre, dans le document [Nahorniak 2003], les spectres sont acquis à haute résolution spectrale d'excitation, puis moyennés, ce qui est de nature à réduire l'erreur de prédiction. Au contraire, dans le cas de l'invention, seulement deux à six spectres sont acquis, avec une résolution spectrale en excitation extrêmement faible, inférieure à 20 nm/pixel voire à 50 nm/pixel. Malgré cela, un choix opportun des paramètres du modèle multivoies et/ou un prétraitement opportun des données permettent de limiter l'erreur de prédiction à des valeurs tout à fait acceptables, typiquement inférieures à 15% voire à 10%.

Un procédé selon l'invention peut comporter également une phase préliminaire d'étalonnage comportant :

i) l'éclairage d'une pluralité d'échantillons d'étalonnage par lesdits rayonnements lumineux d'excitation ;

ii) l'acquisition des spectres de fluorescence frontale des échantillons, correspondant auxdits rayonnements lumineux d'excitation;

iii) un prétraitement des spectres de fluorescence acquis ;

iv) la détermination, par une méthode itérative, desdits vecteurs de *loadings* d'excitation et d'émission du modèle multivoies, ainsi que d'un vecteur de scores pour chaque échantillon d'étalonnage ; et

v) la détermination d'une fonction de régression liant lesdits vecteurs de scores aux valeurs, connues, dudit ou de chaque paramètre pour lesdits échantillons d'étalonnage.

Les spectres de fluorescence peuvent être des spectres d'autofluorescence ou, dans certains cas, des spectres de fluorescence induite par un marqueur ajouté à l'échantillon. Par exemple, l'estimation de la charge microbienne d'un échantillon peut être grandement facilitée par l'utilisation d'une sonde fluorescente.

Avantageusement, les spectres de fluorescence peuvent être prétraités par soustraction d'une contribution due à la diffusion Rayleigh du premier ordre du rayonnement lumineux d'excitation, ladite contribution étant calculée au moyen d'un modèle linéaire généralisé. En particulier, ledit modèle linéaire généralisé peut être déterminé à partir d'une première portion du spectre à prétraiter, comprenant seulement ladite contribution due à la diffusion Rayleigh, et est utilisé pour prédire la contribution de diffusion Rayleigh dans une deuxième portion dudit spectre dans laquelle elle se superpose à une contribution de fluorescence. La soustraction de la diffusion Rayleigh est nécessaire aussi dans les procédés d'analyse connus de l'art antérieur, mais devient critique dans le procédé de l'invention. L'utilisation d'un modèle linéaire généralisé, au lieu des techniques connues de remplacement par des zéros ou des valeurs manquantes, s'avère particulièrement avantageuse lorsqu'un nombre très faible de longueurs d'onde d'excitation est utilisé, comme dans le cas de l'invention.

Ensuite, les spectres de fluorescence peuvent aussi être prétraités en les normalisant. Plusieurs échantillons, d'étalonnage et/ou à analyser, peuvent aussi être prétraités en effectuant une correction multiplicative de dispersion (MSC : « multiplicative scatter correction »).

Selon un mode de réalisation préféré de l'invention, ledit modèle statistique multivoies peut être un modèle PARAFAC, les spectres de fluorescence de chaque échantillon étant représentés sous forme concaténée. Dans ce cas, les vecteurs de *loadings* dudit modèle PARAFAC peuvent être déterminés par une méthode itérative qui est arrêtée lorsque la diminution de la fonction de perte due à la dernière itération descend au-dessous d'une valeur de seuil (« paramètre de convergence ») comprise entre $10^{-4}$ et $10^{-2}$. Cette valeur doit être comparée à celle utilisée généralement de $10^{-6}$. En effet, l'utilisation d'un paramètre de convergence plus grand facilite la convergence du modèle PARAFAC lorsqu'un faible nombre de longueurs d'onde d'excitation est utilisé.

En variante, ledit modèle statistique multivoies peut être un modèle NPLS, les spectres de fluorescence de chaque échantillon étant représentés sous forme concaténée. Dans ce cas, les spectres de fluorescence peuvent utilement être prétraités par correction orthogonale du signal.

Avantageusement, lesdits rayonnements lumineux d'excitation peuvent comprendre : un premier rayonnement à une longueur d'onde comprise entre 270 et 300 nm ; un deuxième rayonnement à une longueur d'onde comprise entre 300 et 360 nm ; et un troisième rayonnement à une longueur d'onde comprise entre 400 et 500 nm.

Ledit ou au moins un dit paramètre à déterminer peut être choisi, notamment, parmi : la teneur dudit ou de chaque échantillons en une substance d'intérêt ; une charge microbienne dudit ou de chaque échantillon ; et une valeur stérilisatrice, pasteurisatrice ou cuisatrice, ou un produit temps-température, d'un procédé auquel ledit ou chaque échantillon a été soumis.

Les notions de valeur stérilisatrice, pasteurisatrice ou cuisatrice sont bien connues par elles-mêmes. On considère une étape d'un procédé thermique au cours de laquelle l'échantillon est maintenu à une température $T_i$ (en °C, à coeur de l'échantillon) pendant un temps $t_i$. La valeur de pasteurisation de cette étape vaut :

$$VP_i = t_i \times 10^{\frac{T_i - 70}{Z}}$$

sa valeur stérilisatrice vaut :

$$VS_i = t_i \times 10^{\frac{T_i - 121,1}{Z}}$$

et sa valeur cuisatrice vaut :

$$VC_i = t_i \times 10^{\frac{T_i - 100}{Z}}$$

où le paramètre Z dépend du microorganisme considéré (pour les valeurs pasteurisatrice et stérilisatrice) ou de la nature de l'échantillon (pour la valeur cuisatrice).

Pour un procédé multi-étapes on prend la somme des valeurs pasteurisatrices ou stérilisatrices des différentes étapes.

Les échantillons à analyser et d'étalonnage peuvent être, notamment, des échantillons d'un produit choisi parmi un aliment et un médicament.

Un autre objet de l'invention est l'utilisation d'un procédé tel que décrit ci-dessus pour mesurer l'évolution des propriétés nutritionnelles, microbiologiques et/ou toxicologiques d'un aliment au cours de sa préparation ou conservation.

Encore un autre objet de l'invention est un appareil d'analyse spectroscopique selon la revendication 13. De préférence un tel appareil peut comporter entre deux et six (et de préférence entre trois et cinq) desdites sources lumineuses.

Avantageusement, un tel appareil peut comporter : une première source lumineuse émettant un rayonnement à une longueur d'onde comprise entre 270 et 300 nm ; une deuxième source lumineuse émettant un rayonnement à une longueur d'onde comprise entre 300 et 360 nm ; et une troisième source lumineuse émettant un rayonnement à une longueur d'onde comprise entre 400 et 500 nm.

D'autres caractéristiques, détails et avantages de l'invention ressortiront à la lecture de la description faite en référence aux dessins annexés donnés à titre d'exemple et qui représentent, respectivement :

- La figure 1, trois spectres « bruts » de fluorescence d'un échantillon de chicorée torréfiée, correspondant à trois rayonnements d'excitation à des longueurs d'ondes différentes ;
- Les figures 2A, 2B, 2C, trois graphiques illustrant l'opération de soustraction de la diffusion de Rayleigh par la méthode utilisant un modèle linéaire généralisé ;
- Les figures 3A, 3B, 3C, trois graphiques comparant ladite méthode de soustraction de la diffusion de Rayleigh (figure 3C) à deux autres méthodes connues de l'art antérieur : le remplacement par des zéros (figure 3A) et le remplacement par des valeurs manquantes (figure 3B) ;
- La figure 4, une illustration schématique de la notion de concaténation des spectres ;
- La figure 5, une illustration schématique de la méthode PARAFAC;
- La figure 6, le schéma de principe d'un appareil d'analyse selon un mode de réalisation de l'invention ;
- Les figures 7 et 8, des graphiques illustrant l'application d'une méthode selon un premier mode de réalisation de l'invention, basée sur un modèle PARAFAC, à la prédiction de la teneur en acrylamide de différents échantillons de chicorée ;
- Les figures 9A, 9B, 10A et 10B, des graphiques illustrant l'application d'une méthode selon un deuxième mode de réalisation de l'invention, basée sur un modèle NPLS, à la prédiction de la teneur en vitamine C de différents échantillons de haricots verts.

Le procédé de l'invention utilise le signal de fluorescence émis à la surface de l'aliment après illumination par des faisceaux lumineux à des longueurs d'onde déterminées du domaine UV-visible (approximativement : 250 - 750 nm). Ce signal est analysé par des méthodes chimiométriques qui permettent d'extraire l'information qui se trouve corrélée aux paramètres que l'on cherche à mesurer. L'existence d'une telle corrélation est déduite du fait que, au cours de la production agricole, la conservation et la transformation, la fluorescence intrinsèque des constituants naturels de l'aliment (vitamines, protéines et autres constituants naturels ou ajoutés intentionnellement ou non), ainsi que leur réflectance, évoluent, alors que dans le même temps, de nouveaux signaux apparaissent du fait de la formation de nouvelles molécules (dont les contaminants néoformés). On parle de fluorescence néoformée ou acquise selon que les fluorophores sont formés de novo ou proviennent de l'environnement. L'évolution conjointe des signaux natifs (SN), néoformés (SNF) et nouvellement acquis (SNA) est corrélée de manière robuste aux modifications physiques, physico-chimiques, chimiques ou microbiologiques de l'aliment, en particulier aux changements des paramètres de qualité, induits au cours de la production, la conservation et/ou la transformation. Les facteurs de changement qui influent sur la qualité de l'aliment sont les rayonnements ultraviolets oxydants, la destruction de microorganismes ou, au contraire, le développement de certains d'entre eux pouvant entraîner la synthèse de mycotoxines, l'intervention humaine sur les cultures (engrais, pesticides ...), ou l'application de procédés modifiant la température, la pression ou tout autre paramètre physique au sein de l'aliment et qui induisent par voie de conséquence une modification de la composition physico-chimique et des paramètres de qualité.

Les rayonnements lumineux d'excitation présentent des longueurs d'onde choisies de manière à explorer le spectre UV-visible le plus largement possible. De manière générale, on peut choisir a priori :

- une longueur d'onde comprise entre 270 et 300 nm permettant d'exciter le tryptophane, les phénols comme l'acide chlorogénique ou l'hydroxytyrosol ou encore la vitamine E, molécules émettant dans l'UV ;
- une longueur d'onde comprise entre 400 et 450 ou 500 nm permettant d'exciter la riboflavine, les porphyrines et la chlorophylle, molécules émettant dans le visible (500-700 nm) ;
- une ou deux longueurs d'onde comprises entre 300 et 360 voire 400 nm sont introduites pour exciter les fluorophores néoformés, essentiellement des produits de Maillard et des produits de peroxydation lipidique, mais également les mycotoxines émettant dans l'UV lointain-proche visible (400-500 nm).

Ces longueurs d'onde seront modifiées en cas d'applications autres qu'à l'industrie alimentaire. En tout cas, pour plus de précision, il est possible de choisir les longueurs d'onde les plus proches possibles des maxima des loadings représentant le vecteur excitation obtenu par la décomposition PARAFAC d'une matrice MEE complète obtenue avec un fluorimètre de laboratoire sur d'un lot d'échantillons représentatifs.

Le nombre de longueurs d'onde utilisées est compris entre 2 et 6, avantageusement entre 3 et 5, et de préférence égal à 5, permettant d'exciter tour à tour un nombre correspondant de groupes de fluorophores parmi ceux décrits plus haut. L'intensité du rayonnement d'excitation est choisie de sorte à ce que l'énergie d'émission de fluorescence de ces fluorophores soit significativement modifiée au cours des étapes de transformation que l'on cherche à caractériser. Par exemple, l'intensité doit être suffisante pour que l'apparition de mycotoxines liée au développement de champignons sur une graine au cours de sa transformation modifie sensiblement l'émission de fluorescence recueillie sur le détecteur à partir d'un échantillon non contaminé. Elle doit également permettre de mettre en évidence l'application de tel traitement thermique caractérisé par un couple temps-température ; ou encore l'application d'un lavage, blanchiment ou traitement de décontamination.

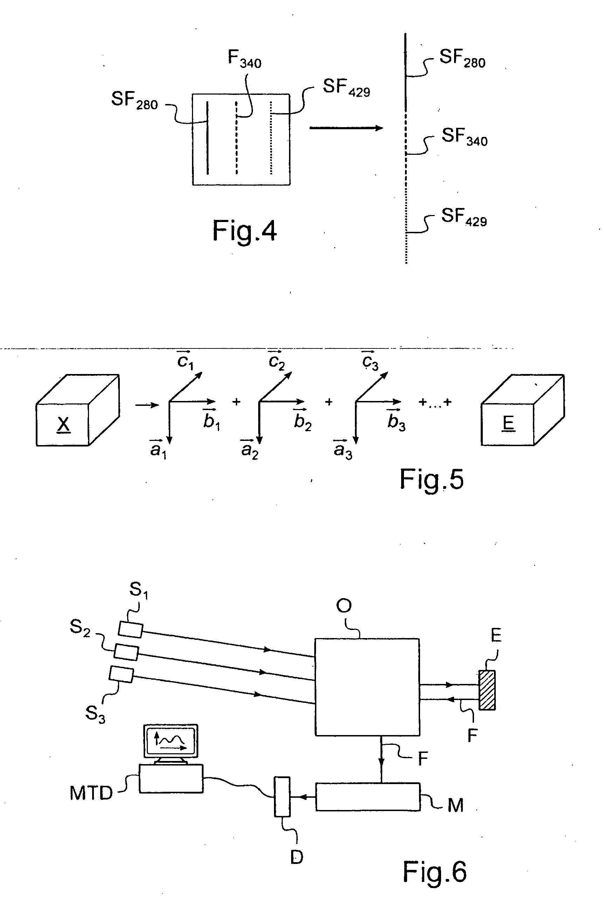

La figure 6 montre un schéma très simplifié d'un appareil pour la mise en oeuvre du procédé de l'invention. Cet appareil comprend trois sources lumineuses $S_1$, $S_2$, $S_3$ émettant chacune un faisceau de rayonnement monochromatique à une longueur d'onde différente dirigée de sorte à éclairer l'échantillon E. Le signal de fluorescence F (en fait, mélange de fluorescence et diffusion Rayleigh du 1er et parfois du 2nd ordre) émis par cet échantillon est transporté par une fibre optique vers un spectromètre qui décompose le rayonnement lumineux émis en spectre. Les spectres acquis sont traités par un moyen de traitement des données MTD (typiquement un ordinateur programmé d'une manière opportune) qui permet d'en extraire l'information chimiométrique recherchée.

Les sources lumineuses peuvent être, typiquement, des diodes électroluminescentes, voire des lasers (de préférence à semiconducteur) si des intensités plus importantes sont requises.

L'analyse des données spectroscopiques, effectuée par l'ordinateur MTD, comporte quatre étapes principales :

- le prétraitement des spectres ;
- la détermination des vecteurs des *«loadings »* du modèle multivoies à l'aide des spectres des échantillons d'étalonnage ;
- la détermination des vecteurs des « scores » en appliquant ce modèle aux spectres des échantillons à analyser ; et
- la détermination d'une information de nature chimique (teneur en une ou plusieurs substances d'intérêt), physique (produit temps-température, valeur stérilisatrice, pasteurisatrice ou cuisatrice), physico-chimique ou microbiologique (charge microbienne) à partir desdits vecteurs des « *scores* ».

On considère d'abord le prétraitement des spectres, en référence à un exemple spécifique, dans lequel un échantillon de chicorée torréfiée est éclairé successivement par trois rayonnements d'excitation à 280, 340 et 429 nm. Chacun des trois spectres de fluorescence est constitué de 1515 valeurs d'intensité spectrale pour autant de longueurs d'ondes λ différentes. La résolution spectrale est de 0,25 nm/pixel, mais elle peut être divisée par 5, voire plus, sans qu'une dégradation des résultats du procédé de l'invention ne soit observée.

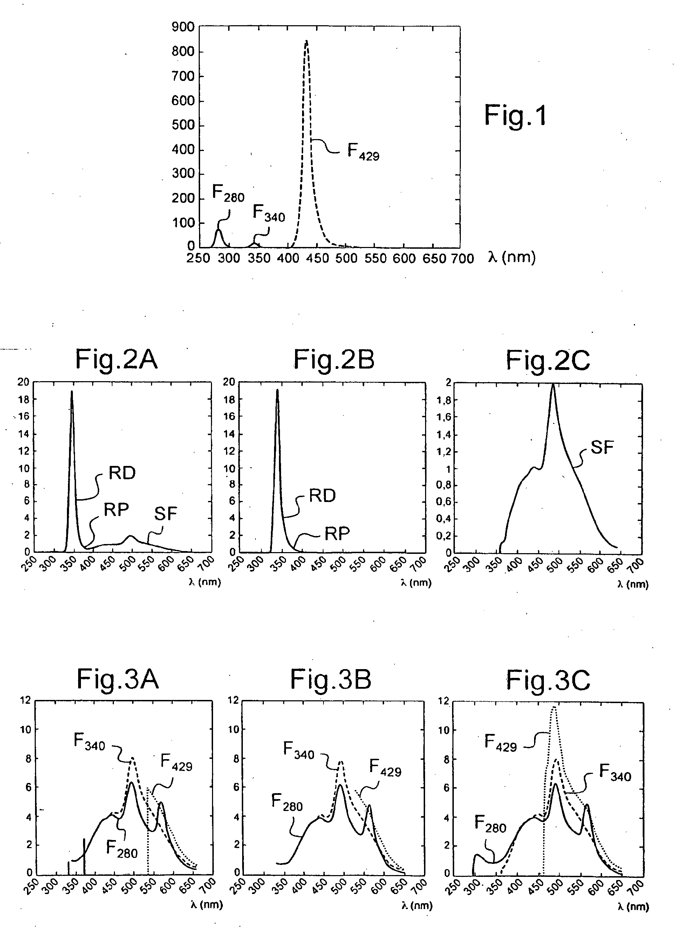

Les spectres « bruts » (figure 1) sont dominés par la diffusion de Rayleigh du premier ordre, à la longueur d'onde des rayonnements d'excitation (280, 340 et 429 nm). Le spectre de chaque signal de fluorescence, en effet, est partiellement superposé au rayonnement d'excitation diffusé, qui est beaucoup plus intense. Ce problème est connu, et est généralement résolu en remplaçant les valeurs d'intensité correspondant à la région spectrale de superposition par des zéros (figure 3A) ou des valeurs manquantes (figure 3B). Sur ces figures les références $F_{280}$, $F_{340}$ et $F_{429}$ représentent les spectres de fluorescence relatifs aux excitations à 280, 340 et 429 nm respectivement, dans lesquels les intensités des régions spectrales de chevauchement avec la diffusion de Rayleigh ont été remplacées par des zéros ou des valeurs manquantes.

Ces méthodes présentent des inconvénients, car le remplacement par des zéros engendre des artéfacts et donc fausse l'analyse en créant des variances artificielles. Le remplacement par valeurs manquantes engendre des difficultés au niveau de la convergence des modèles multivoies utilisés pour l'analyse des données. De plus, la présence des valeurs manquantes empêche l'utilisation des prétraitements de standardisation (voir ci-après), qui ne peuvent être appliqués qu'à des valeurs réelles.

Ces problèmes sont acceptables dans le cas des techniques conventionnelles, basées sur l'utilisation de MEE de grandes dimensions, mais deviennent facilement rédhibitoires dans un procédé tel que celui de l'invention, basé sur l'analyse de données très faiblement résolues en longueur d'onde d'excitation.

Pour cette raison, l'invention propose de supprimer la contribution de la diffusion de Rayleigh du premier ordre par une technique innovante qui se base sur la prédiction de la région de diffusion qui chevauche la fluorescence via un modèle linéaire généralisé (GLZ) avec une fonction de liaison log [Davidson 1998 ; Rizkallah 2007]. Ce modèle est calibré sur une région du spectre dans laquelle la contribution de la fluorescence est négligeable, et l'intensité spectrale est attribuée exclusivement à la diffusion (référence RD sur les figures 2A et 2B).

Le modèle linéaire généralisé est de la forme:

$$f(\mu_y) = b_0 + b_1 \mathbf{x}$$

Dans l'équation, $f(\mu_y)$ est la fonction de liaison de $\mu_y$, valeur attendue de y, y étant le vecteur des intensités de diffusion de Rayleigh qui ne se chevauchent pas avec la fluorescence, tandis que x est un vecteur d'indices (1,2,3, etc.) de même taille que y.

Grace au modèle généralisé, des relations non linéaires (entre x et y) peuvent être modélisées via la fonction de liaison. Le modèle généralisé peut être utilisé pour modéliser des variables dépendantes ayant des distributions appartenant à la famille exponentielle (Normale, Gamma, Poisson, etc.). La régression linéaire multiple est un cas spécial du modèle GLZ qui correspond à une fonction de liaison égale à la fonction identité et à une variable dépendante (y) présentant une distribution normale.

Les paramètres $b_i$ du modèle GLZ sont estimés par la méthode statistique de maximisation de la vraisemblance (L) :

$$L = F(Y, \text{modèle}) = \prod_{i=1}^{n} p[y_i, b_i].$$

$p[y_i, b_i]$ étant la probabilité de $y_i$ conditionnée par $b_i$.

Le but est trouver les paramètres qui donnent la plus grande probabilité (densité jointe) de réalisation de y pour toutes les observations. Une estimation itérative (algorithme de Fisher, qui est une méthode quasi-Newtonienne) est utilisée pour trouver les paramètres $b_i$ en maximisant L:

$$\frac{\partial \log(L)}{\partial b_i} = 0$$

Une fois les $b_i$ estimés, le modèle GLZ est appliqué sur les indices de la diffusion correspondant à la région spectrale superposée avec la fluorescence (fig2B - région de prédiction RP) pour prédire les intensités de la diffusion « pure ». Après cette prédiction, les spectres de diffusion complets (partie réelle et prédite) sont soustraits des MEE pour obtenir le spectre de fluorescence pure SF (figure 2C - à comparer avec la figure 2A montrant le spectre de fluorescence partiellement superposé au spectre de la lumière diffusée).

Les opérations successives ne portent plus sur les trois spectres de fluorescence considérés individuellement, mais sur les spectres concaténés, c'est à dire disposés l'un à la suite de l'autre dans une colonne unique (voir la figure 4).

Les spectres concaténés peuvent être soumis selon les cas à :

- Une simple normalisation par division de chaque valeur d'émission de chaque spectre concaténé, par la norme de ce même vecteur $X_i$. Cette normalisation est appliquée à chaque échantillon (i=1 à I échantillons), soit :

$$x_{ij(\text{normalisé})} = \frac{x_{ij}}{\|x_{i.}\|}$$

où j est le nombre de longueurs d'ondes du spectre d'émission concaténés (j= 1 à 4545) et $\|x_{i.}\| = \sqrt{\sum_j |x_{ij}|^2}$ ; ou

- Une correction par « *Multiplicative scatter correction* » (correction multiplicative de la dispersion) qui consiste à réaliser une régression entre le vecteur $x_i$ (le spectre d'émission concaténé de chaque échantillon i) et le vecteur concaténé moyen de tous les échantillons (i=1 à I échantillons), puis à retrancher l'ordonnée à l'origine et à diviser par la pente respectives chaque intensité du spectre concaténé d'émission pour chaque échantillon, comme décrit ci-dessous:

$$x_{i.} = a_i + b_i \overline{x}_{i.}$$

$$x_{ij}(MSC) = \frac{x_{ij} - a_i}{b_i}$$

Voir à ce propos : [Bro 1998 ; Dhanoa 1994]

Après avoir acquis et prétraité les spectres de fluorescence d'un certain nombre d'échantillons d'étalonnage, il est possible de déterminer le modèle statistique multivoies qui sera utilisé pour l'analyse d'autres échantillons, à caractériser. Cela comporte le calcul des vecteurs de *« loadings »* de ce modèle.

On considère d'abord le cas d'un modèle multilinéaire de type « PARAFAC » [Bro 1998]. Le principe, illustré par la figure 5, est de décomposer une structure à trois voies (cube de données) X en une somme de produits externes de trois vecteurs (« triades ») $a_i$, $b_i$, $c_i$, plus un résidu E, lui aussi sous la forme d'un « cube de données ». Les trois voies constituant le « cube » X sont : les échantillons, les rayonnements d'excitation, les longueurs d'onde d'émission. On peut donc écrire :

$$x_{ijk} = \sum_{f=1}^{F} a_{if} \, b_{jf} \, c_{kf} + e_{ijk} \, ,$$

Où : « i » est l'index des échantillons, « j » celui des rayonnements d'excitation, « k » celui des longueurs d'onde d'émission, « f » celui des F facteurs de décomposition PARAFAC.

La concaténation des spectres permet d'écrire la décomposition PARAFAC sous forme matricielle :

$$\mathbf{X_{I^*JK} = A(C \otimes B)^T};$$

où :

- I, J et K sont les nombres d'échantillons, de longueurs d'onde d'excitation et de longueurs d'onde d'émission respectivement ;
- $X_{I^*JK}$ est la matrice des fluorescences concaténées de tous les échantillons ;
- $B_{JF}$ et $C_{KF}$ sont les vecteurs (à JF et KF éléments, respectivement) *« loadings »* d'excitation et d'émission (profils bilinéaires de fluorescence) ;
- $A_{IF}$ est le vecteur (à IF éléments) des « *scores* » (ou intensités des *loadings* spectraux) ;
- le symbole ⊗ représente le produit tensoriel de Khatri-Rao ; et
- l'exposant T indique la transposée de la matrice colonne.

Les vecteurs des *loadings* sont calculés à partir d'échantillons d'étalonnage choisis de manière à être représentatifs de la variabilité totale attendue au sein du groupe des échantillons à prédire subséquemment. En d'autres termes, le modèle est empirique ; il n'est donc valide que pour des échantillons similaires à ceux qui ont servi à l'étalonnage.

Les paramètres A, B et C du modèle PARAFAC peuvent être calculés par la méthode d'alternance des moindres carrés (méthode itérative non linéaire). Dans cette méthode une première estimation du vecteur A est calculée conditionnellement sur des valeurs initiales aléatoires attribuées à B et C pour minimiser la somme des carrés des résidus. Le paramètre B est ensuite actualisé en utilisant l'estimation de A, puis le paramètre C est actualisé en utilisant la nouvelle valeur de B ; et ainsi de suite. Chaque actualisation itérative de A, B et C améliore donc la solution (baisse de la surface d'erreur). L'algorithme converge lorsque l'amélioration de la solution au niveau d'une itération devient très petite (par défaut, ce critère est de $10^{-6}$).

La convergence du modèle PARAFAC pour des données faiblement résolues et qui, de plus, ne sont pas véritablement trilinéaires, peut être problématique [Bro 1998 ; Harshman 1984 ; Rizkallah 2007]. En effet, la surface d'erreur du modèle peut contenir des minima locaux (points plus bas que leur voisinage mais plus élevés que le vrai minimum de la surface), des points col, des terrains plats, et des vallées très étroites qui peuvent ralentir ou bien empêcher la convergence de l'algorithme.

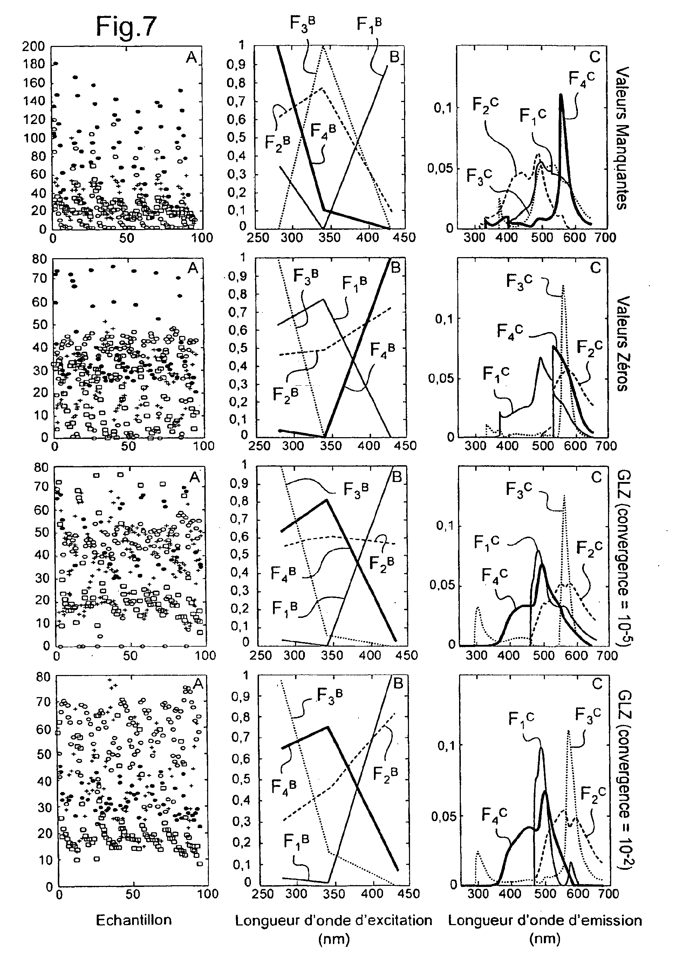

Les présents inventeurs ont remarqué qu'en imposant une limitation sur le nombre d'itérations (par exemple 30 itérations maximum) et/ou en augmentant le critère de convergence ($10^{-2}$ ou $10^{-3}$ au lieu de $10^{-6}$) on améliore significativement le modèle au niveau des paramètres (*loadings* et scores), comme cela sera montré ci-après à l'aide de la figure 7. En effet, un nombre excessif d'itérations peut dégrader la pertinence du modèle.

La convergence peut être facilitée en imposant une contrainte de non négativité puisqu'on sait a priori que les spectres de fluorescence ainsi que leurs intensités relatives ne peuvent pas prendre des valeurs négatives.

Enfin, il a été remarqué que le prétraitement de suppression de la diffusion de Rayleigh par application d'un modèle GLZ facilite la convergence du procédé itératif de calcul des vecteurs de *loadings* et de *scores* du modèle PARAFAC.

Dans le cas de données parfaitement multilinéaires, le modèle PARAFAC n'admet en théorie qu'une solution unique. Cependant, dans le cas d'une MEE limitée à 3 excitations, et lorsque la fluorescence est recueillie en surface, la trilinéarité de la matrice MEE est fortement perturbée.

De ce fait, la solution n'est plus unique et il est nécessaire de développer des critères de choix du nombre de facteurs ainsi que du modèle final.

Ce choix est guidé par plusieurs critères connus en soi [Bro 1998 ; Harshman1984 ] :

- La vérification de la conformité entre les paramètres spectraux obtenus (vecteurs B et C) et la connaissance a priori des fluorophores présents dans l'échantillon analysé.
- Un critère connu sous le nom de CORCONDIA [Bro 1998] qui sert à vérifier le pourcentage de déviation du modèle par rapport à un modèle multi-linéarité parfait.
- L'étude du pourcentage de variance des données de fluorescence expliqué par le modèle.
- L'étude de la structure des résidus, qui doivent être aléatoires.
- L'étude de la structure des *scores* (vecteur A) en termes de cohérence de l'évolution des scores par rapport à ce qui est attendu de l'application de la transformation sur le lot d'échantillons d'étalonnage.
- La répétabilité et la reproductibilité des *scores,* etc.

Les *scores* ainsi obtenus par la décomposition PARAFAC des MEE des échantillons d'étalonnage sont ensuite utilisés pour construire une régression multiple (linéaire ou généralisée) sur les concentrations du ou des paramètres de qualité à mesurer. Les coefficients de la régression ainsi que les vecteurs B et C sont stockés dans une base de données du modèle.

Le modèle est validé par application à de nouveaux échantillons connus pour lesquels les valeurs prédites sont comparées aux valeurs mesurées par les méthodes de référence.

Les MEE obtenues sur de nouveaux échantillons sont prétraités (élimination de Rayleigh et standardisation) de la même façon que les MEE des échantillons d'étalonnage. Le modèle PARAFAC et le modèle de régression sont appliqués sur les nouveaux échantillons en utilisant les coefficients stockés (B, C et les coefficients de régression) obtenus à partir des échantillons d'étalonnage. Dans le cas de l'utilisation d'une normalisation de type MSC, le spectre moyen des échantillons d'étalonnage fournit la référence pour corriger les spectres des nouveaux échantillons.

A partir du cube des matrices prétraitées des nouveaux échantillons à analyser, les nouveaux scores sont calculés sur la base des vecteurs « *loadings* » B et C comme suit :

$$A_{nouveau} = (B \otimes C)^{+} * X_{nouveau} \; ;$$

où l'exposant + indique l'inverse généralisé du produit tensoriel et $X_{nouveau}$ les données spectrales concaténées et prétraitées des nouveaux échantillons. Les nouveaux scores sont enfin multipliés par les coefficients de régression (obtenus sur les échantillons d'étalonnage) pour obtenir les paramètres de qualité des nouveaux échantillons.

Ainsi les opérations nécessaires pour prédire les concentrations des paramètres de qualité dans les échantillons nouveaux à analyser consistent à :

- prétraiter les nouveaux spectres (éliminer la diffusion et standardiser) ;
- calculer les scores des échantillons à analyser à partir des vecteurs B et C en mémoire ; et
- appliquer l'équation de régression en mémoire pour obtenir les concentrations des paramètres.

La méthode décrite ci-dessus à été appliquée pour prédire la teneur en acrylamide dans des échantillons de chicorée (n=68).

Pour chaque échantillon, trois spectres ont été acquis avec des excitations à 280, 340 et 429 nm, prétraités par suppression de la diffusion et standardisation, et concaténés ; puis les MEE ainsi obtenues ont été décomposées en quatre facteurs PARAFAC.

Sur la figure 7 :

- les graphiques de la première colonne, libellés « A », représentent les « *scores* » des quatre facteurs PARAFAC (respectivement : points noirs, cercles gris, carrés, étoiles) pour 100 échantillons ;
- les graphiques de la deuxième colonne, libellés « B » représentent les « *loadings* » d'excitation des quatre facteurs PARAFAC (courbes $F^B_1$, $F^B_2$, $F^B_3$, $F^B_4$) pour ces mêmes 100 échantillons ;
- les graphiques de la troisième colonne, libellés « C » représentent les « *loadings* » d'émission des quatre facteurs PARAFAC (courbes $F^C_1$, $F^C_2$, $F^C_3$, $F^C_4$) pour ces mêmes 100 échantillons.

Les graphiques de la première ligne se rapportent au cas où la diffusion de Rayleigh a été supprimée par introduction de valeurs manquantes ; ceux de la troisième ligne, au cas où elle a été supprimée par introduction de zéros ; ceux de la troisième et quatrième ligne, au cas où la suppression de la diffusion de Rayleigh a été effectuée en utilisant un modèle linéaire généralisé.

Á l'exception des données qui incluent des valeurs manquantes, toutes les autres données ont été aussi standardisées par MSC.

Les résultats montrent que les valeurs manquantes au niveau du chevauchement de la diffusion et de la fluorescence (fig. 7C ligne 1) sont mal prédites tandis que les zéros produisent des artéfacts (fig. 7C ligne 2) constitués par des variations brusques d'intensité. Les modèles prétraités avec GLZ produisent des paramètres spectraux acceptables, avec des variations d'intensité graduelles (fig. 7C lignes 3 et 4).

Le critère de convergence du modèle de la figure 7 lignes 1, 2 et 3 est de $10^{-6}$ (critère communément utilisé) ; celui du modèle de la figure 7 ligne 4 est de $10^{-2}$. La restriction imposée sur la convergence du modèle soit par limitation du nombre d'itérations permissible et/ou en augmentant le critère de convergence améliore très significativement les résultats modèle en termes de structure des scores (fig. 7A lignes 3 et 4) et de séparation des facteurs (fig. 7B et C lignes 3 et 4). En particulier, on peut remarquer l'évolution de la courbe $F^B_2$ qui, dans la troisième ligne de la figure, montre une contribution des trois longueurs d'onde d'excitation ; au contraire, dans la quatrième ligne, la séparation des excitations est nettement meilleure. De même, l'épaulement de la courbe $F^C_1$ est réduit considérablement lorsque le facteur de convergence passe de $10^{-6}$ à $10^{-2}$.

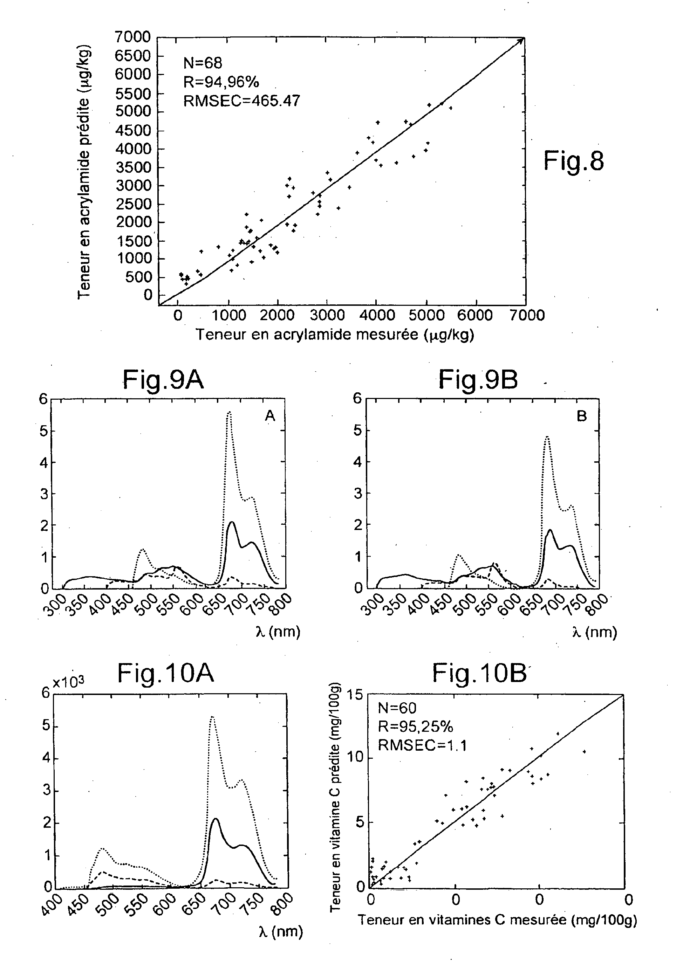

Les scores issus du modèle présenté dans la figure 7, ligne 4 ont été utilisés pour calibrer la teneur en acrylamide chimiquement mesurée dans les échantillons. Les teneurs en acrylamide chimiquement mesurées et prédites par le modèle basé sur les scores PARAFAC de la fluorescence mesurée sur le capteur sont présentés dans la figure 8. Sur cette figure, les teneurs en acrylamide mesurées conventionnellement sont reportées sur l'axe des abscisses, celles prédites par le modèle le sont sur l'axe des ordonnées. N est le nombre d'échantillons, R est le coefficient de corrélation et le RMSEC est la racine carré de la moyenne des carrés des erreurs. On remarquera en particulier la valeur élevée du coefficient de corrélation (presque 95%) ; la RMSEC vaut 465,47 $\mu$g/kg, c'est à dire environ 15% de la valeur moyenne de la teneur en acrylamide mesurée.

Quand la régression issue des scores PARAFAC de fluorescence n'aboutit pas à des résultats convenables (par exemple coefficient de corrélation R < 0,85), une autre stratégie qui vise à améliorer la prédiction du modèle de régression est appliquée. Cette méthode se base sur la régression multivoies NPLS, de préférence couplée à un prétraitement par Correction Orthogonale du Signal (OSC) [Wold 1998].

Le prétraitement OSC vise à réduire ou éliminer la variance contenue dans le signal de fluorescence qui est orthogonale (non corrélée) à la variable dépendante (y) avant d'exécuter la régression. L'OSC est appliquée sur les données concaténées après élimination de la diffusion par la méthode GLZ et prétraitement de standardisation par MSC.

L'algorithme le plus fréquemment utilisé pour la correction OSC est le NIPALS (« non linear iterative partial least squares », c'est à dire algorithme non linéaire et itérative par moindres carrés partiels). La première étape consiste à extraire le vecteur des *scores* **t** de là première composante principale (décomposition en valeurs singulières) et l'orthogonaliser sur le vecteur **y** : $\mathbf{t}_{nouveau} = (1 - \mathbf{y}(\mathbf{y}^T\mathbf{y})^{-1}\mathbf{y}^T)\,\mathbf{t}$. Ensuite, une régression type MLR ou PLS (multivariée) est appliquée pour prédire le vecteur orthogonalisé : $\mathbf{w} = \mathbf{X}^+ \mathbf{t}_{nouveau}$ (la convergence est testée sur le nouveau score $\mathbf{t}^* = \mathbf{Xw}$). Lorsque t* converge, le vecteur propre correspondant est calculé $\mathbf{p} = \mathbf{X}^T \mathbf{t}^*$ et l'effet orthogonal est ensuite éliminé des données de fluorescence ($\mathbf{X}_{nouveau} = \mathbf{X} - \mathbf{t}^*\mathbf{p}^T$). Dans l'application à l'invention, un seul facteur OSC est calculé puis éliminé avant la réorganisation des données de fluorescence en une structure trois voies. Dans le cas d'une seule variable **y,** le modèle converge en une seul itération.

Contrairement au modèle de décomposition PARAFAC, le modèle de régression NPLS [Bro 1998] cherche des facteurs explicatifs à la fois des données de fluorescence <u>**X**</u> (trois voies) et de **y** (la variable dépendante). La régression NPLS cherche donc une décomposition des données <u>**X**</u> qui aboutit à des scores qui ont une covariance maximale avec **y** ; une régression interne entre les scores NPLS de **X** et **y** aboutit ensuite à l'étalonnage

$$X^{(I \times JK)} = T(W^K \otimes W^J)^T + E_1 \quad ;$$

$$y = Tb + E_2 \quad ;$$

où T est le vecteur des *scores* de **X** (ayant la meilleur covariance avec **y**) et les vecteurs *loadings* respectives pour les excitations et les émissions sont **$W^K$ et $W^J$**.

L'algorithme pour la NPLS avec f facteurs correspondant à des données **X** (trois voies) et une seule variable dépendante est décrit comme suit :

1. Centrer les données **X** et **y** et on pose $y_0 = y$
2. Commencer l'itération f=1
3. Calculer $Z = X^T y$
4. Déterminer $w^J$ et $w^K$ en décomposant z en valeurs singulières
5. Calculer les vecteurs **t** et les arranger dans une matrice T= $[t_1 \ t_2 \ t_3 \ t_f]$
6. Calculer les coefficients de régression $b = (T^T T)^{-1} T^T y_0$
7. Chaque échantillon $X_i$ est remplacé par $X_i - t_i w^J (w^K)^T$ et $y = y_0 - Tb$
8. $f = f + 1$. Redémarrer de la phase 1 jusqu'à explication suffisante de **y**.

(Dans ces équations, l'indice du facteur f a été omis de $t, w^J, w^K,$ et **b**)

Etant donné que la NPLS est appliquée sur les données après prétraitement OSC (donc la majeur variance perpendiculaire à y a été éliminée) un seul facteur NPLS est utilisé pour la régression.

Après concaténation des MEE des nouveaux échantillons, le prétraitement MSC est appliqué en utilisant le spectre moyen des échantillons d'étalonnage comme référence. Les facteurs OSC (**p et w**) calculés sur les échantillons d'étalonnage sont ensuite éliminés des nouveaux échantillons :

$$X_c = X_n - X_n * w * (p^T * w)^{-1} * p^T;$$

$X_n$ est la matrice des MEE concaténées et prétraitées par MSC et $X_c$ la matrice des MEE concaténées corrigées par OSC.

Les loadings ($W^J, W^K$) et les coefficients de régression **b** de la NPLS obtenus à partir des échantillons d'étalonnage sont appliqués sur la matrice $X_c$ pour prédire les scores des nouveaux échantillons et leur variable dépendante.

$$T_n = (W^J \otimes W^K)^+ * X_c$$

$$\hat{y} = T_n b + e.$$

La méthode NPLS a été appliquée à la prédiction de la teneur en vitamine C dans des échantillons de haricots verts.

Les figures 9A, 9B, 10A et 10B, des graphiques illustrant l'application d'une méthode selon un deuxième mode de réalisation de l'invention, basée sur un modèle N-PLS, à la prédiction de la teneur en vitamine C de différents échantillons de haricots verts.

Après acquisition des matrices MEE de n=60 échantillons d'haricots verts (figure 9A : spectres bruts), élimination de la diffusion (modèle GLZ) et standardisation par MSC, un facteur OSC a été appliqué pour éliminer des données fluorescence la majeur partie de la variance orthogonale (non explicative) à la variable dépendante (teneur en vitamine C chimiquement mesurée) : voir figure 9B, spectres prétraités.

Ensuite, un modèle NPLS avec un facteur a été appliqué pour calibrer le « cube » des MEE prétraités sur la teneur en vitamine C chimiquement mesurée.

La figure 10A montre les coefficients de régression NPLS pour les trois spectres d'émission (excitation à 280nm : ligne noire ; excitation à 340nm : ligne grise; excitation à 429 nm : ligne discontinue). La figure 10B montre un graphique des teneurs en vitamine C mesurées conventionnellement (axe des abscisses) et prédites par application du modèle NPLS à trois spectres d'autofluorescence (axe des ordonnées). N est le nombre d'échantillons, R est le coefficient de corrélation et le RMSEC est la racine carré de la moyenne des carrés des erreurs. Là encore on remarquera la valeur très élevée du coefficient de corrélation (supérieure à 95%) et le fait que l'erreur quadratique moyenne RMSEC est de l'ordre de 15%, acceptable pour les applications considérées ici.

L'invention a été décrite en référence à deux exemples particuliers dans lesquels elle a été appliquée à la détermination de la teneur d'échantillons alimentaires en une substance d'intérêt. L'invention n'est cependant pas limitée à ce type d'applications. Plus généralement, elle peut permettre la détermination de différents indicateurs de qualité d'un échantillon ; ces indicateurs peuvent être de type chimique, comme dans les exemples précités, mais également de type microbiologique (charge microbienne), physico-chimique ou chimique. L'invention permet aussi de caractériser les procédés auxquels des échantillons sont soumis, en procurant des estimations de paramètres tels que la valeur stérilisatrice ou pasteurisatrice, le produit temps-température ou la valeur cuisatrice d'un procédé de traitement thermique.

Références :

[Bro 1998] Bro, R. « Multi-way Analysis in the Food Industry Models, Algorithms, and Applications », PhD thesis, Universiteit van Amsterdam, 1998.

[Dhanoa 1994] Dhanoa, M.S.; Lister, S.J.; Sanderson, R.; Barnes, R.J. « The link between multiplicative scatter correction (MSC) and standard normal variate (SNV) transformations of NIR spectra. » J. Near Infrared Spectrosc. 1994, 2, 43-47.

[Harshman 1984] Harshman, R. A. « « How can I know if it's real? » A catalog of diagnostics to use with three-mode factor analysis and multidimensional scaling. » In: Research methods for multimode data analysis. Law, H.G.; Snyder, C.W.; Hattie, J.A.; McDonald, R.P., eds. pp. 566-571, Praeger, New York, 1984.

[Rizkallah 2007] Rizkallah, J. « Chemometric Analysis of Front Face Fluorescence Data - Estimation, of Neoformed Contaminants in Processed Foods. » PhD thesis, Agro Paris Tech France, 2007.

[Wold 1998] Wold, S.; Antti, H.; Lindgren, F.; Öhman. « Orthogonal signal correction of near-infrared spectra. » Chemometr. Intell. Lab. Syst. 1998, 44, 175-185.

[Nahorniak 2003] Nahorniak Michelle L., Booksh Karl S. « Optimizing the implementation of the PARAFAC method for near-real time calibration of excitation-emission fluorescence analysis » J. Chemometrics 2003; 17; 608-617.

[Rizkallah et al., 2008] Rizkallah J., Lakhal L., Birlouez-Aragon I.: « Front face fluorescence to monitor food processing and neoformed contamination » In « Optical Methods for Monitoring Fresh and Processed Food - Basics and Applications for a better Understanding of Non-Destructive Sensing » Chapter 4. Editor Zude M. Publisher, CRC Press, USA (2008)

[Davidson 1998] Davidson, R. ; MacKinnon J.G. « Econometric theory and methods. Generalized least squares and related methods » in « Econometric Theory and Methods », Oxford University Press 2004, pp 255-261.

[Christensen 2005] Christensen J., Miquel Becker E., Fredriksen C.S. "Fluorescence spectroscopy and PARAFAC in the analysis of yogurt", CHEMOMETRICS AND INTELLIGENT LABORATORY SYSTEMS, ELSEVIER SCIENCE PUBLISHERS B.V. AMSTERDAM, NL, vol. 75, no. 2, 28 février 2005 (2005-02-28), pages 201-208, ISSN: 0169-7439

**Revendications**

1. Procédé d'analyse spectroscopique d'au moins un échantillon mettant en oeuvre une méthode d'analyse des données spectroscopiques basée sur un modèle statistique multivoies, le procédé comportant :

   a) l'éclairage dudit ou de chaque échantillon à analyser (E) par une pluralité de rayonnements lumineux d'excitation à des longueurs d'onde respectives ;
   b) l'acquisition de spectres de fluorescence frontale ($F_{280}$, $F_{340}$, $F_{429}$) dudit ou de chaque échantillon, correspondant chacun à un rayonnement lumineux d'excitation respectif ;
   c) un prétraitement des spectres de fluorescence acquis ;
   d) pour chaque échantillon, le calcul d'un vecteur de *scores* par application aux spectres prétraités dudit modèle statistique multivoies, identifié par un vecteur de *loadings* d'excitation de la fluorescence et par un vecteur de *loadings* d'émission ; et

e) la détermination d'au moins un paramètre choisi parmi un indicateur de qualité dudit ou de chaque échantillon et un paramètre caractérisant un procédé auquel ledit ou chaque échantillon a été soumis, à partir dudit vecteur des *scores ;*

le procédé étant **caractérisé en ce que** l'écart spectral moyen entre lesdits rayonnements lumineux d'excitation est d'au moins 50 nm, sur une plage spectrale d'au moins 100 nm.

2. Procédé selon la revendication 1 dans lequel le nombre de rayonnements lumineux d'excitation, et de spectres de fluorescence correspondants pour chaque échantillon, est compris entre deux et six, et de préférence entre 3 et 5, sur une plage spectrale d'au moins 100 nm, et de préférence d'au moins 150 nm.

3. Procédé selon l'une des revendications précédentes, comprenant également une phase préliminaire d'étalonnage comportant :

   i) l'éclairage d'une pluralité d'échantillons d'étalonnage par lesdits rayonnements lumineux d'excitation ;
   ii) l'acquisition des spectres de fluorescence frontale des échantillons, correspondant auxdits rayonnements lumineux d'excitation ;
   iii) un prétraitement des spectres de fluorescence acquis ;
   iv) la détermination, par une méthode itérative, desdits vecteurs des *loadings* d'excitation et d'émission du modèle multivoies, ainsi que d'un vecteur de *scores* pour chaque échantillon d'étalonnage ; et
   v) la détermination d'une fonction de régression liant lesdits vecteurs de scores aux valeurs, connues, dudit ou de chaque paramètre pour lesdits échantillons d'étalonnage.

4. Procédé selon l'une des revendications précédentes, dans lequel les spectres de fluorescence sont prétraités par soustraction d'une contribution due à la diffusion Rayleigh du premier ordre du rayonnement lumineux d'excitation, ladite contribution étant calculée au moyen d'un modèle linéaire généralisé.

5. Procédé selon la revendication 4, dans lequel ledit modèle linéaire généralisé est déterminé à partir d'une première portion (RD) du spectre à prétraiter, comprenant seulement ladite contribution due à la diffusion Rayleigh, et est utilisé pour prédire la contribution de diffusion Rayleigh dans une deuxième portion (RP) dudit spectre dans laquelle elle se superpose à une contribution de fluorescence.

6. Procédé selon l'une des revendications précédentes, dans lequel ledit modèle statistique multivoies est un modèle PARAFAC, les spectres de fluorescence de chaque échantillon étant représentés sous forme concaténée.

7. Procédé selon la revendication 6 dans lequel les vecteurs de *loadings* dudit modèle PARAFAC sont déterminés par une méthode itérative qui est arrêtée lorsque la diminution de la fonction de perte due à la dernière itération descend au-dessous d'une valeur de seuil comprise entre $10^{-4}$ et $10^{-2}$.

8. Procédé selon l'une des revendications 1 à 5, dans lequel ledit modèle statistique multivoies est un modèle NPLS, les spectres de fluorescence de chaque échantillon étant représentés sous forme concaténée.

9. Procédé selon l'une des revendications précédentes dans lequel lesdits rayonnements lumineux d'excitation comprennent :

   - un premier rayonnement à une longueur d'onde comprise entre 270 et 300 nm ;
   - un deuxième rayonnement à une longueur d'onde comprise entre 300 et 360 nm ; et
   - un troisième rayonnement à une longueur d'onde comprise entre 400 et 500 nm.

10. Procédé selon l'une des revendications précédentes dans lequel ledit ou au moins un dit paramètre à déterminer est choisi parmi :

    - la teneur dudit ou de chaque échantillon en une substance d'intérêt ;
    - une charge microbienne dudit ou de chaque échantillon ; et
    - une valeur stérilisatrice, pasteurisatrice ou cuisatrice, ou un produit temps-température d'un procédé auquel ledit ou chaque échantillon a été soumis.

11. Procédé selon l'une des revendications précédentes dans lequel les échantillons à analyser et d'étalonnage sont des échantillons d'un produit choisi parmi : un aliment et un médicament.

**12.** Utilisation d'un procédé selon la revendication 11 pour mesurer l'évolution des propriétés nutritionnelles, microbiologiques et/ou toxicologiques d'un aliment au cours de sa préparation ou conservation.

**13.** Appareil d'analyse spectroscopique d'au moins un échantillon comportant :

- un ensemble de sources lumineuses ($S_1$, $S_2$, $S_3$) pour éclairer ledit ou chaque échantillon à analyser (E) par des rayonnements lumineux d'excitation respectifs, présentant des longueurs d'onde différentes avec un écart spectral moyen d'au moins 50 nm sur une plage spectrale d'au moins 100 nm ;
- un moyen d'acquisition (M, D) des spectres de fluorescence frontale émis par ledit ou de chaque échantillon lorsqu'il est éclairé par lesdits rayonnements lumineux d'excitation ; et
- un moyen de traitement (MTD) des spectres de fluorescence acquis, adapté pour mettre en oeuvre un procédé selon l'une des revendications 1 à 11.

**14.** Appareil d'analyse spectroscopique selon la revendication 13 comportant entre deux et six, et de préférence entre trois et cinq, desdites sources lumineuses.

**15.** Appareil selon la revendication 14 comportant :

- une première source lumineuse ($S_1$) émettant un rayonnement à une longueur d'onde comprise entre 270 et 300 nm ;
- une deuxième source lumineuse ($S_2$) émettant un rayonnement à une longueur d'onde comprise entre 300 et 360 nm ; et
- une troisième source lumineuse ($S_3$) émettant un rayonnement à une longueur d'onde comprise entre 400 et 500 nm.

## Patentansprüche

**1.** Verfahren zur spektroskopischen Analyse wenigstens einer Probe unter Einsatz eines Verfahrens zur Analyse von spektroskopischen Daten basierend auf einem Multikanal-Statistikmodel, wobei das Verfahren umfasst:

a) Beleuchtung der oder jeder zu analysierenden Probe (E) durch eine Vielzahl von Anregungslichtbestrahlungen mit entsprechenden Wellenlängen;
b) Erfassen von frontalen Fluoreszenzspektren ($F_{280}$, $F_{340}$, $F4_{29}$) der oder jeder Probe, jeweils in Entsprechung zu einer entsprechenden Anregungslichtbestrahlung;
c) eine Vorverarbeitung der erfassten Fluoreszenzspektren;
d) für jede Probe, die Berechung eines Scores-Vektors durch Anwendung des Multikanal-Statistikmodels auf die vorverarbeiteten Spektren, identifiziert durch einen Anregungs-Loadings-Vektor der Fluoreszenz und durch einen Emissions-Loadings-Vektor; und
e) die Bestimmung wenigstens eines Parameters, welcher ausgewählt ist aus einem Indikator einer Qualität der oder jeder Probe und einem Parameter, welcher ein Verfahren charakterisiert, welchem die oder jede Probe unterzogen wurde, ausgehend von dem Scores-Vektor;

wobei das Verfahren **dadurch gekennzeichnet ist, dass** der mittlere spektrale Abstand zwischen den Anregungslichtbestrahlungen wenigstens 50 nm ist, über einen Spektralbereich von wenigstens 100 nm.

**2.** Verfahren nach Anspruch 1, wobei die Anzahl von Anregungslichtbestrahlungen und der entsprechenden Fluoreszenzspektren für jede Probe enthalten ist zwischen zwei und sechs und bevorzugt zwischen drei und fünf, über einen Spektralbereich von wenigstens 100 nm und bevorzugt von wenigstens 150 nm.

**3.** Verfahren nach einem der vorhergehenden Ansprüche, umfassend außerdem eine Kalibrierungs-Vorphase, welche umfasst:

i) Beleuchtung einer Vielzahl von Kalibrierproben durch die Anregungslichtbestrahlungen;
ii) Erfassen von frontalen Fluoreszenzspektren der Proben in Entsprechung zu den Anregungslichtbestrahlungen;

iii) eine Vorverarbeitung der erfassten Fluoreszenzspektren;

iv) die Bestimmung, über ein iteratives Verfahren, von Anregungs- und Emissions-Loadings-Vektoren des Multikanalmodels, sowie eines Scores-Vektors für jede Kalibrierprobe; und

v) die Bestimmung einer Regressionsfunktion, welche die Scores-Vektoren mit den bekannten Werten der oder jedes Parameters für die Kalibrierproben verknüpft.

4. Verfahren nach einem der vorhergehenden Ansprüche, wobei die Fluoreszenzspektren vorverarbeitet werden durch Subtraktion eines Beitrags aufgrund der Rayleigh-Diffusion erster Ordnung der Anregungslichtbestrahlung, wobei der Beitrag mittels eines generalisierten linearen Models berechnet wird.

5. Verfahren nach Anspruch 4, wobei das generalisierte lineare Model bestimmt wird ausgehend von einem ersten Abschnitt (RD) des vorzuverarbeitenden Spektrums, welcher ausschließlich den Beitrag aufgrund der Rayleigh-Diffusion aufweist, und verwendet wird, um den Beitrag der Rayleigh-Diffusion in einem zweiten Abschnitt (RP) des Spektrums vorherzusagen, in welchem er einem Fluoreszenzbeitrag überlagert ist.

6. Verfahren nach einem der vorhergehenden Ansprüche, wobei das Mulitkanal-Statistikmodel ein PARAFAC-Model ist, wobei die Fluoreszenzspektren jeder Probe in aneinander geketteter Form dargestellt sind.

7. Verfahren nach Anspruch 6, wobei die Loadings-Vektoren des PARAFAC-Models bestimmt sind durch ein iteratives Verfahren, welches angehalten wird, wenn die Verringerung der Verlustfunktion aufgrund der letzten Iteration unterhalb einen Schwellenwert sinkt, welcher zwischen $10^{-4}$ und $10^{-2}$ enthalten ist.

8. Verfahren nach einem der Ansprüche 1 bis 5, wobei das Multikanal-Statistikmodel ein NPLS-Model ist, wobei die Fluoreszenzspektren jeder Probe in aneinander geketteter Form dargestellt sind.

9. Verfahren nach einem der vorhergehenden Ansprüche, wobei die Anregungslichtbestrahlungen umfassen:

   - eine erste Bestrahlung mit einer Wellenlänge, welche zwischen 270 und 300 nm enthalten ist;
   - eine zweite Bestrahlung mit einer Wellenlänge, welche zwischen 300 und 360 nm enthalten ist; und
   - eine dritte Bestrahlung mit einer Wellenlänge, welche zwischen 400 und 500 nm enthalten ist.

10. Verfahren nach einem der vorhergehenden Ansprüche, wobei die oder wenigstens einer der zu bestimmenden Parameter ausgewählt sind aus:

   - der Gehalt der oder jeder Probe an einer Substanz von Interesse;
   - eine mikrobielle Belastung der oder jeder Probe; und
   - ein auf Sterilisation, Pasteurisation oder Kochen bezogener Wert, oder ein Zeit-Temperatur-Produkt eines Verfahrens, welchem die oder jede Probe unterzogen wurde.

11. Verfahren nach einem der vorhergehenden Ansprüche, wobei die zu analysierenden Proben und Kalibrierproben Proben eines Produkts sind, welches ausgewählt ist aus: einem Nahrungsznttel und einem Medikament.

12. Verwendung eines Verfahrens nach Anspruch 11 zur Messung der Entwicklung von nahrungsbezogenen, mikrobiologischen und/oder toxikologischen Eigenschaften eines Nahrungsmittels im Verlauf seiner Herstellung oder Konservierung.

13. Vorrichtung zur spektroskopischen Analyse wenigstens einer Probe, umfassend:

   - eine Anordnung von Lichtquellen ($S_1$, $S_2$, $S_3$) zum Beleuchten der oder jeder zu analysierenden Probe (E) durch entsprechende Anregungslichtbestrahlungen, welche verschiedene Wellenlängen mit einem mittleren spektralen Abstand von wenigstens 50 nm über einen Spektralbereich von wenigstens 100 nm aufweisen;
   - ein Mittel zur Erfassung (M, D) von frontalen Fluoreszenzspektren, welche von der oder jeder Probe emittiert werden, wenn sie durch die Anregungslichtbestrahlungen beleuchtet wird; und
   - ein Mittel zur Verarbeitung (MTD) der erfassten Fluoreszenzspektren, welches dazu ausgestaltet ist, ein Verfahren nach einem der Ansprüche 1 bis 11 einzusetzen.

14. Vorrichtung zur spektroskopischen Anlayse nach Anspruch 13, welche zwischen zwei und sechs und bevorzugt zwischen drei und fünf der Lichtquellen umfasst.

**15.** Vorrichtung nach Anspruch 14, umfassend:

- eine erste Lichtquelle ($S_1$), welche eine Strahlung mit einer Wellenlänge emittiert, welche zwischen 270 und 300 nm enthalten ist;
- eine zweite Lichtquelle ($S_2$), welche eine Strahlung mit einer Wellenlänge emittiert, welche zwischen 300 und 360 nm enthalten ist; und
- eine dritte Lichtquelle ($S_3$), welche eine Strahlung mit einer Wellenlänge emittiert, welche zwischen 400 und 500 nm enthalten ist.

**Claims**

**1.** Method for spectroscopic analysis of at least one sample implementing a method for analysis of spectroscopic data based on a multi-way statistical model, which method includes:

a) the illumination of said or of each sample to be analyzed (E) by a plurality of excitation light radiations with respective wavelengths;

b) the acquisition of front-face fluorescence spectra ($F_{280}$, $F_{340}$, $F_{429}$) of said or of each sample, each corresponding to a respective excitation light radiation;

c) a pre-processing of the fluorescence spectra acquired;

d) for each sample, the calculation of a score vector by application to the pre-processed spectra of said multi-way statistical model, identified by a fluorescence excitation loading vector and by an emission loading vector; and

e) the determination of at least one parameter chosen from a quality indicator of said or of each sample and a parameter characterizing a method to which said or each sample has been subjected, on the basis of said score vector;

the method being **characterized in that** the average spectral gap between said excitation light radiations is at least 50 nm over a spectral range of at least 100 nm.

**2.** Method according to claim 1, in which the number of excitation light radiations and corresponding fluorescence spectra for each sample is between two and six, and preferably between 3 and 5, over a spectral range of at least 100 nm, and preferably of at least 150 nm.

**3.** Method according to one of the previous claims, also including a preliminary calibration phase comprising:

i) the illumination of a plurality of calibration samples by said excitation light radiations;

ii) the acquisition of front-face fluorescence spectra of the samples, corresponding to said excitation light radiations;

iii) a pre-processing of the acquired fluorescence spectra;

iv) the determination, by an iterative method, of said loading excitation and emission vectors of the multi-way model, as well as a score vector for each calibration sample; and

v) the determination of a regression function linking said scores to the known values of said or of each parameter for said calibration samples.

**4.** Method according to one of the previous claims, in which the fluorescence spectra are pre-processed by subtracting a contribution due to the first-order Rayleigh scattering of the excitation light radiation, with said contribution being calculated by means of a generalized linear model.

**5.** Method according to claim 4, in which said generalized linear model is determined on the basis of a first portion (RD) of the spectrum to be pre-processed, including only said contribution due to the Rayleigh scattering, and is used to predict the Rayleigh scattering contribution in a second portion (RP) of said spectrum in which it is superimposed with a fluorescence contribution.

**6.** Method according to one of the previous claims, in which said multi-way statistical model is a PARAFAC model, with the fluorescence spectra of each sample being represented in concatenated form.

**7.** Method according to claim 6, in which the loading vectors of said PARAFAC model are determined by an iterative method that is stopped when the reduction of the loss function due to the last iteration goes below a threshold value

between $10^{-4}$ and $10^{-2}$.

8. Method according to one of claims 1 to 5, in which said multi-way statistical model is an NPLS model, with the fluorescence spectra of each sample being represented in concatenated form.

9. Method according to one of the previous claims, in which said excitation light radiation includes:

   - a first radiation with a wavelength of between 270 and 300 nm;
   - a second radiation with a wavelength of between 300 and 360 nm; and
   - a third radiation with a wavelength of between 400 and 500 nm.

10. Method according to one of the previous claims, in which said or at least one said parameter to be determined is chosen from:

    - the content of a substance of interest in said or in each sample;
    - a microbial load of said or of each sample; and
    - a sterilizing, pasteurizing or cooking value, or a time-temperature product, of a method to which said or each sample has been subjected.

11. Method according to one of the previous claims, in which the samples to be analyzed and for calibration are samples of a product chosen from: a food and a drug.

12. Use of a method according to claim 11 to measure the change in nutritional, microbiological and/or toxicological properties of a food during its preparation or preservation.

13. Appliance for spectroscopic analysis of at least one sample comprising:

    - a set of light sources ($S_1$, $S_2$, $S_3$) for illuminating said or each sample to be analyzed (E) by respective excitation light radiations, having different wavelengths with an average spectral gap of at least 50 nm over a spectral range of at least 100 nm;
    - means for acquisition (M, D) of the front-face fluorescence spectra emitted by said or each sample when illuminated by said excitation light radiations, and
    - means for processing the fluorescence spectra acquired, suitable for implementing a method according to one of claims 1 to 11.

14. Appliance for spectroscopic analysis according to claim 13, comprising between two and six, and preferably between three and five, of said light sources.

15. Appliance according to claim 14, comprising:

    - a first light source ($S_1$) emitting radiation at a wavelength of between 270 and 300 nm;
    - a second light source ($S_2$) emitting radiation at a wavelength of between 300 and 360 nm; and
    - a third light source ($S_3$) emitting radiation at a wavelength of between 400 and 500 nm.

Fig.1

Fig.2A

Fig.2B

Fig.2C

Fig.3A

Fig.3B

Fig.3C

Fig.4

Fig.5

Fig.6

# Fig.7

Fig.8

Fig.9A

Fig.9B

Fig.10A

Fig.10B

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **BRO, R.** Multi-way Analysis in the Food Industry Models, Algorithms, and Applications. *PhD thesis,* 1998
- **DHANOA, M.S. ; LISTER, S.J. ; SANDERSON, R ; BARNES, R.J.** The link between multiplicative scatter correction (MSC) and standard normal variate (SNV) transformations of NIR spectra. *J. Near Infrared Spectrosc,* 1994, vol. 2, 43-47
- « « How can I know if it's real? » A catalog of diagnostics to use with three-mode factor analysis and multidimensional scaling. ». **HARSHMAN, R. A.** Research methods for multimode data analysis. 1984, 566-571
- **RIZKALLAH, J.** Chemometric Analysis of Front Face Fluorescence Data - Estimation, of Neoformed Contaminants in Processed Foods. *PhD thesis,* 2007
- **WOLD, S. ; ANTTI, H. ; LINDGREN ; F.; ÖHMAN.** Orthogonal signal correction of near-infrared spectra. *Chemometr. Intell. Lab. Syst.,* 1998, vol. 44, 175-185

- **NAHORNIAK MICHELLE L. ; BOOKSH KARL S.** Optimizing the implementation of the PARAFAC method for near-real time calibration of excitation-emission fluorescence analysis. *J. Chemometrics,* 2003, vol. 17, 608-617
- Front face fluorescence to monitor food processing and neoformed contamination. **RIZKALLAH J. ; LAKHAL L. ; BIRLOUEZ-ARAGON I.** Optical Methods for Monitoring Fresh and Processed Food - Basics and Applications for a better Understanding of Non-Destructive Sensing. CRC Press, 2008
- Econometric theory and methods. Generalized least squares and related methods. **DAVIDSON, R. ; MACKINNON J.G.** Econometric Theory and Methods. Oxford University Press, 2004, 255-261
- Fluorescence spectroscopy and PARAFAC in the analysis of yogurt. **CHRISTENSEN J. ; MIQUEL BECKER E. ; FREDRIKSEN C.S.** CHEMOMETRICS AND INTELLIGENT LABORATORY SYSTEMS. ELSEVIER SCIENCE PUBLISHERS, 28 Février 2005, vol. 75, 201-208